(19) Europäisches Patentamt
European Patent Office
Office européen des brevets

(11) Publication number: **0 410 551 A1**

(12) # EUROPEAN PATENT APPLICATION

(21) Application number: **90250188.1**

(51) Int. Cl.5: **C07D 285/125**, A01N 43/82

(22) Date of filing: **24.07.90**

(30) Priority: **26.07.89 DE 3925176**

(43) Date of publication of application: **30.01.91 Bulletin 91/05**

(84) Designated Contracting States: **AT BE CH DE DK ES FR GB GR IT LI LU NL SE**

(71) Applicant: **SCHERING AKTIENGESELLSCHAFT**
**Müllerstrasse 170/178**
**D-1000 Berlin 65(DE)**

(72) Inventor: **Hübl, Dieter, Dr.**
**Käthe-Kollwitz-Strasse 123**
**D-4712 Werne(DE)**
Inventor: **Pieroh, Ernst**
**Holme 25**
**D-2263 Risum-Lindholm(DE)**
Inventor: **Joppien, Hartmut, Dr.**
**Juttastrasse 18**
**D-1000 Berlin 37(DE)**

(54) **5-Substituted 1,3,4-thiadiazole derivatives, their preparation and their use as pesticides.**

(57) 5-Substituted 1,3,4-thiadiazole derivatives of general formula I

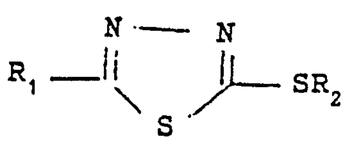

wherein
$R_1$ is $C_{1-14}$-alkyl, $C_{3-6}$-cycloalkyl or $C_{3-6}$-cycloalkylmethyl, and
$R_2$ is $C_{1-12}$-alkyl, $C_{2-12}$-alkenyl, $C_{2-12}$-alkynyl, $C_{3-6}$-cycloalkyl or $C_{3-6}$-cycloalkylmethyl, each of which is substituted one or more times by the same or different halogen, processes for their preparation and their use as pesticides, especially against nematodes.

EP 0 410 551 A1

# 5-SUBSTITUTED 1,3,4-THIADIAZOLE DERIVATIVES, THEIR PREPARATION AND THEIR USE AS PESTICIDES.

This invention relates to new 5-substituted 1,3,4-thiadiazole derivatives, their preparation as well as their use as pesticides, especially against plant parasitic nematodes.

1,3,4-Thiadiazoles with nematicidal activity are already known, eg as in WO 86/7590. This specification discloses various heterocycles, including a 1,3,4-thiadiazole, carrying a haloalkenylthio group. In such thiadiazoles, the haloalkenylthio is in the 2-position and groups that are mentioned that can be the 5-position are aryl, arylalkyl, aryloxyalkyl or various thio or amino groups. We have now found that thiadiazoles carrying different substituents in the 5-position on the thiadiazole have good nematicidal activity. As well as haloalkenylthio other thio groups can also be present in the 2-position.

Thus the invention provides 5-substituted 1,3,4-thiadiazole derivatives of general formula I

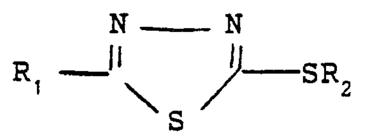

(I)

wherein

$R_1$ is $C_{1-14}$-alkyl, $C_{3-6}$-cycloalkyl or $C_{3-6}$-cycloalkylmethyl, and

$R_2$ is $C_{1-12}$-alkyl, $C_{2-12}$-alkenyl, $C_{2-12}$-alkynyl, $C_{3-6}$-cycloalkyl or $C_{3-6}$-cycloalkylmethyl, each of which is substituted one or more times by the same or different halogen.

These compounds show a surprisingly good activity against nematodes, especially plant parasitic nematodes, coupled with good plant compatibility.

The compounds of the invention also have good activity against biting and sucking insects and their eggs and also against acarids.

In a preferred group of compounds

$R_1$ is $C_{1-4}$-alkyl, especially methyl, or $C_{3-6}$-cycloalkyl, especially cyclopropyl and

$R_2$ is halo-$C_{1-4}$-alkyl, eg halomethyl and especially difluoromethyl or bromodifluoromethyl, or halo-$C_{2-4}$-alkenyl, eg fluoro-$C_{2-4}$-alkenyl, and especially 3,3,4-trifluoro-3-butenyl.

The compounds of the invention of general formula I can be prepared according to known methods by reacting a compound of general formula II,

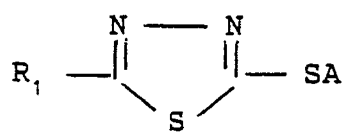

(II)

in which A is hydrogen, ammonium or an alkali metal and $R_1$ has the meaning given in formula I, with a compound of formula Z-$R_2$, in which Z is a leaving group, such as for example halogen, mesylate or tosylate, and $R_2$ has the meaning given above, in an inert solvent or solvent mixture, optionally at raised temperature and optionally at raised pressure, in the presence of a base.

Suitable bases include organic and inorganic bases, such as for example tertiary amines, eg triethylamine or tripropylamine, alkali metal and alkaline earth metal hydrides, hydroxides, carbonates and bicarbonates and also alkali metal alcoholates, such as sodium methoxide or potassium tert.-butylate.

Suitable solvents for the preparation of the compounds of the invention include for example diethyl ether, dioxane and tetrahydrofuran; aliphatic and aromatic hydrocarbons, such as toluene and petroleum ether; halogenated hydrocarbons, eg chlorobenzene, methylene chloride, carbon tetrachloride and chloroform; nitriles such as acetonitrile and propionitrile; N,N-dialkylamides, such as dimethylformamide; ketones, such as acetone and methyl ethyl ketone; dimethyl sulphoxide, sulpholane, as well as water and alcohols, eg methanol, ethanol, isopropanol or butanol, and mixtures of such solvents.

The reaction temperature depends on the reactants and can vary between -70 °C and 120 °C. The pressure also depends on the reactants and can lie between 1 and 25 bar. The reaction usually lasts from ca. 0.5 to 48 hours. The reaction mixture can be poured into ice/water, extracted and worked up in known manner. The resulting products can be purified in conventional manner, for example by recrystallisation,

vacuum distillation or column chromatography.

The compounds of formula II used as starting material are either known or can be obtained in an analogous way to known processes (EP 217 747, DE 2 541 115 and J. Heterocyclic Chem. 19 , 541 (1982)).

Because of the nematicidal activity coupled with good plant compatibility, the compounds according to the invention can be successfully applied in plant protection as pesticides in agriculture, in vine and fruit growing, in horticulture and in forestry.

Plant parasitic nematodes which can be controlled according to the invention include for example root-knot nematodes, such as Meloidogyne incognita , Meloidogyne hapla and Meloidogyne javanica , cyst forming nematodes, such as Globodera rostochiensis , Heterodera schacktii , Heterodera avanae , Heterodera glycines and Heterodera trifolii , and stem and leaf eelworms, such as Ditylenchus dipsaci , Ditylenchus destructor , Aphelenchoides ritzemabosi , Pratylenchus neglectus , Pratylenchus penetrans , Pratylenchus curvitatus , as well as Tylenchorhynchus dubius , Tylenchorhynchus claytoni , Rotylenchus robustus , Heliocotylenchus multicinctus , Radopholus similis , Belonolaimus longicaudatus , Longidorus elongatus and Trichodorus primitivus .

Based on their insecticidal and acaricidal properties, the compounds of the invention further offer the possibility of treatments against pests in the different stages of crops as well as human and animal pests.

The use of the active ingredients of the invention can be carried out in the form of their conventional commercial formulations and/or the ready to use preparations from these formulations.

The content of active ingredient in the ready to use preparations obtained from the commercial concentrated formulations can vary over wide ranges. The rate of use for the control of nematodes lies between 0.03 kg to around 10 kg per hectare, preferably from around 0.3 kg to around 6 kg per hectare.

The active ingredient or their mixtures can be applied in the usual formulations such as solutions, emulsions, wettable powders, suspensions, powders, dusts, foams, pastes, soluble powders, granules, aerosols, suspension concentrates, seed dressings, natural and synthetic substances impregnated with the active ingredients, microcapsules in polymers and in seed coatings for seeds, as well as formulations with burning substances such as smoke cartridges, smoke capsules and smoke spirals amongst others as well as ULV-cold and hot fogging formulations.

These formulations can be prepared in known manner for example by mixing the active ingredient with diluents such as liquid solvents, and liquefied gases and/or solid carriers, optionally using surface active agents such as emulsifiers and/or dispersing agents and/or foaming agents.

When using water as the diluent, organic solvents can also be used for example as auxiliary solvents.

Examples of liquid solvents include aromatic hydrocarbons, such as xylene, toluene or alkynaphthalenes, chlorinated aromatic or chlorinated aliphatic hydrocarbons, such as chlorobenzene, chloroethylene or methylene dichloride, aliphatic hydrocarbons, such as cyclohexane or paraffins, for example mineral oil fractions, alcohols, such as butanol and glycol as well as their ethers and esters, ketones, such as acetone, methyl ethyl ketone, methyl isobutyl ketone or cyclohexanone, strongly polar solvents, such as dimethylformamide and dimethyl sulphoxide, as well as water.

By the term liquefied gaseous diluents or carriers are meant those substances which are gaseous at normal temperature and pressure, for example aerosol blowing agents, such as halohydrocarbons, as well as butane, propane, nitrogen and carbon dioxide.

Examples of solid carriers are natural earth powders, such as kaolin, alumina, talc, chalk, quartz, attapulgite, montmorillonite or diatomaceous earths and synthetic powders, such as finely divided silica, aluminium oxide and silicates as well as solid carriers for granules, crushed and fractionated natural rocks, such as calcite, marble, pumice, sepiolith and dolomite, as well as synthetic granules from inorganic and organic powders as well as granules from organic materials such as sawdust, coconut shells, maize cobs and tobacco stalks.

Examples of emulsifying and/or foaming agents include non-ionic and anionic emulsifiers, such as polyoxyethylene-fatty acid esters, polyoxyethylene-fatty alcohol ethers, for example alkylaryl-polyglycolethers, alkylsulphonates and arylsulphonates as well as protein hydrolysates.

Dispersing agents include for example lignin, sulphite waste liquors and methylcellulose.

There can also be used in the formulations sticking agents such as carboxymethylcellulose, natural and synthetic powdery, granulated or latex-forming polymers, as well as gum arabic, polyvinyl alcohol and polyvinyl acetate.

There can also be used dyestuffs, such as inorganic pigments, for example iron oxide, titanium oxide, ferrocyan blue and organic dyestuffs such as alizarin- and azo-metal phthalocyanine dyestuffs and trace elements such as salts of iron, manganese, boron, copper, cobalt, molybdenum and zinc.

The formulations contain in general between 0.1 and 95 weight percent of the active ingredient, preferably between 0.5 and 90 percent.

Examples of formulations are:

I. Wettable powder

10 parts by weight of the compound of Example 1 were intimately mixed with 12 parts by weight of calcium lignosulphonate, 76 parts by weight of finely divided kaolin and 2 parts by weight of dialkylnaphthalene sulphonate and then milled.

II. Dusting powder

2.5 parts by weight of the compound of Example 1 were dissolved in 10 methylene chloride and added to a mixture of 25 parts by weight of finely divided silicic acid and 71.5 parts by weight talc and 1 part by weight sudan red. The solvent was removed in vacuo and the residue finely milled.

III. Granule

5 parts by weight of the weight of the compound of Example 1 were dissolved in 10 parts by weight of methylene chloride and sprayed onto 95 parts by weight granulated attapulgite of particle size 0.3 -0.8 mm and dried.

IV. Emulsifiable concentrate

20 parts by weight of the compound of Example 1 were dissolved in a mixture of 75 parts by weight of isophorone and 5 parts by weight of a mixture of 30 parts by weight of calcium benzene sulphonate and 30 parts by weight of castor oil polyglycolate with 40 mole % ethylene oxide and 40 parts by weight of a copolymer of propylene- and ethylene oxide.

The following Examples illustrate the preparation of compounds of the invention.

Example 1

5-Cyclopropyl-2-difluoromethylthio-1,3,4-thiadiazole

16.93 g (0.107 mol) 5-Cyclopropyl-1,3,4-thiadiazole-2-thiol was suspended in 100 ml dioxane and treated with a solution of 30.0 g potassium hydroxide in 58 ml water. At a temperature of 70 °C, a steady stream of chlorodifluoromethane was passed through the mixture over 60 minutes. It was then cooled and poured into 500 ml ice/water and extracted three times with 100 ml ethyl acetate. The organic phase was dried over magnesium sulphate, filtered and concentrated. The residual oil was purified by column chromatography. (Eluent: Hexane/ethyl acetate 4:1)

Yield: 6.17 g = 27.7% of theory

$n_D^{20}$: 1.5472

Example 2

5-Cyclopropyl-2-(3,4,4-trifluoro-3-butenylthio-1,3,4-thiadiazole

4.7 g (0.158 mol) of an 80% suspension of sodium hydride in paraffin oil, which had been washed with 40 ml toluene, was suspended in 20 ml dimethylformamide. At a temperature of 20 °C, a solution of 20 g (0.126 mol) 5-cyclopropyl-1,3,4-thiadiazole-2-thiol in 100 ml dimethylformamide was added dropwise and the mixture stirred for 15 minutes. 32.76 g (0.158 mol) 4-Bromo-1,1,2-trifluorobut-1-ene was added dropwise and the mixture stirred for 3 hours at room temperature. The mixture was added to 500 ml ice/water and extracted three times with 100 ml ethyl acetate. The organic phase was dried over magnesium sulphate, filtered and concentrated. The residual oil was purified by column chromatography (eluent: hexane/ethyl acetate 8:2).

Yield: 10.97 g = 32.7% of theory

$n^{20}_D$: 1.5265

In a similar manner, the following compounds of formula I were prepared.

| Example No | $R_1$ | $R_2$ | mp(°C)/$n_D^{20}$ |
|---|---|---|---|
| 3 | 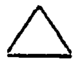 | $CF_2Br$ | 1,5603 |
| 4 | t-but | $CF_2H$ | 44 |
| 5 | i-but | $CF_2Br$ | 1,5267 |
| 6 | t-but | $CF_2Br$ | 45 |
| 7 | i-$C_4H_9$ | $CF_2$=CF-$CH_2CH_2$ | 1,4989 |
| 8 | n-$C_3H_7$ | $CF_2$=CF-$CH_2CH_2$ | 1,5043 |
| 9 | n-$C_3H_7$ | $CHF_2$ | 1,5096 |
| 10 | n-$C_3H_7$ | $CF_2Br$ | 1,5403 |
| 11 | t-$C_4H_9$ | $CF_2$=CF-$CH_2CH_2$ | 1,4949 |
| 12 | 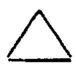 | ClCH=CH-$CH_2$ | 1,5953 |
| 13 | 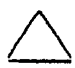 | $CH_2$=CCl-$CH_2$ | 1,6000 |
| 14 | $CH_3$ | $CF_2$=CF-$CH_2CH_2$ | 1,5155 |
| 15 | $CH_3$ | $CF_2Br$ | 53-55 |
| 16 | $CH_3$ | $CHF_2$ | 1,5293 |
| 17 | n-$C_5H_{11}$ | $CF_2$=CF-$CH_2CH_2$ | 1,5045 |
| 18 | n-$C_5H_{11}$ | $CF_2Br$ | 1,5277 |
| 19 | n-$C_5H_{11}$ | $CHF_2$ | 1,5045 |
| 20 | i-$C_3H_7$ | $CF_2$=CF-$CH_2CH_2$ | 1,5123 |
| 21 | i-$C_3H_7$ | $CF_2Br$ | 1,5305 |
| 22 | i-$C_3H_7$ | $CHF_2$ | 1,5140 |
| 23 | s-$C_4H_9$ | $CF_2$=CF-$CH_2CH_2$ | 1,5023 |
| 24 | s-$C_4H_9$ | $CHF_2$ | 1,5036 |
| 25 | $C_2H_5$ | $CF_2$=CF-$CH_2CH_2$ | 1,5104 |

| Example No | $R_1$ | $R_2$ | mp(°C)/$n_D^{20}$ |
|---|---|---|---|
| 26 | $C_2H_5$ | $CHF_2$ | 1,5192 |
| 27 | $C_2H_5$ | $CF_2Br$ | 1,5471 |
| 28 | $n-C_4H_9$ | $CF_2=CF-CH_2CH_2$ | 1,5021 |
| 29 | $n-C_6H_{13}$ | $CF_2=CF-CH_2CH_2$ | 1,4982 |
| 30 | $n-C_6H_{13}$ | $CHF_2$ | 1,4987 |
| 31 | $n-C_6H_{13}$ | $CF_2Br$ | 1,5222 |
| 32 | $n-C_4H_9$ | $CHF_2$ | 1,5046 |
| 33 | 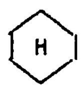 | $CF_2=CF-CH_2CH_2$ | 1,5256 |
| 34 | 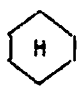 | $CHF_2$ | 1,5352 |
| 35 | 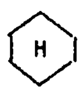 | $CF_2Br$ | 1,5542 |
| 36 | 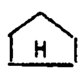 | $CHF_2$ | 1,5378 |
| 37 | 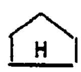 | $CF_2=CF-CH_2CH_2$ | 1,5271 |

The following use examples illustrate the biological activity of the compounds of the invention.

Use Example A

Control of root knot nematode, Meloidogyne incognita

5% of a powder preparation of the active ingredient was mixed thoroughly with soil that had been strongly infested with the test nematode. After this the treated soil was put into a 0.5 litre fermenting tube, treated with cucumber seeds and cultivated at a soil temperature of 25 to 27°C in a greenhouse. After a cultivation time of 25 to 28 days the cucumber roots were washed and inspected in a water bath for nematode attack (root knots) and the % level of activity of the active ingredients compared with a treated control was determined. When the nematode attack is fully controlled the level of activity is 100%.

At a dose of 25 mg of active substance per litre of soil, the compounds of Examples 1, 2, 7, 8, 9, 11, 14, 16, 17, 18, 20, 23, 24, 25, 26, 28, 29 and 32 showed 90-100% activity.

Use Example B

Activity in prophylactic treatment of leaves against brown rice-hoppers (Nilaparvata lugens Stal)

In a heated greenhouse, rice seedlings (about 15 per pot) were grown until formation of the third leaf and then sprayed until dripping wet with an aqueous preparation containing 0.1% of active material. After drying the sprayed leaves, a transparent cylinder was placed over each pot. 30 Adult brown rice-hoppers ( Nilaparvata lugens ) were introduced into each pot. After 2 days at 25-26 °C in the greenhouse, the amount of dead hoppers was determined. The activity was calculated according to Abbott in comparison with several untreated control pots.

The compounds of Examples 1, 4, 5, 8 and 10 showed an activity of 80-100%.

Use Example C

Activity in the prophylactic treatment of feed against the two spotted mite ( Tetranychus urticae Koch )

From the primary leaf of field beans ( Phaseolus vulgaris nanus Aschers .) 14 mm diameter discs were cut. Some of these were treated with a 0.1% aqueous preparations of compounds of the invention and these along side untreated discs were placed on filter papers with the underside of the leaves turned upwards. After drying the test pieces, they were each infested with six adult female Tetranychus urticae and maintained for 3 days at 25 °C and 16 hours light per day. The experiment was replicated 4 times. Dead and alive adults were then counted and removed. Similarly the number of eggs laid were counted. After a further 7 days, the number of living larvae were counted, the activity calculated using Abbott's method in comparison with the untreated controls.

The compounds of Examples 2, 3, 7, 8, 28, 29, 33 and 37 showed 80-100% activity.

Use Example D

Activity against eggs/larvae of the corn rootworm ( Diabrotica undecimpunctata)

The compounds of the invention were made up as aqueous emulsions at a concentration of 0.1%. Into the soil in polystyrene petri dishes, containing maize seedlings (1 seedling/dish) and ca. 50 eggs of the corn rootworm ( Diabrotica undecimpunctata ) were pipetted 0.2 ml of these preparations. The closed dishes were left at 25 °C under extended daylight conditions for 4 days. The criterion for judging the activity was the death of eggs or newly hatched larvae at the end of the test.

The compounds of Examples 1-3, 5-11, 14, 16-20, 23-28 and 30-32 showed 80-100% activity.

Use Example E

Ovicidal activity against eggs of the cotton bollworm ( Heliothis viriscens )

The compounds of the invention were made up as aqueous preparations at a concentration of 0.1%. One day old eggs that had been laid on filter paper by fertilised female moths were dipped in the preparations until they were completely wet and then placed in closed petri dishes under extended daylight conditions for four days at 25 °C. The % inhibition of hatching of the eggs in comparison with untreated eggs indicates the level of activity.

The compound of Examples 1-3, 5-11, 13-20 and 23-32 showed 80-100% activity.

Use Example F

Activity against tick larvae ( Boophilus microplus )

Filter papers (9 cm in diameter) were impregnated with 1 ml aliquots of acetone solutions of test compound at various concentrations. The papers were allowed to dry and then folded into envelopes in

which cattle tick larvae, ( Boophilus microplus ) were enclosed and held at 25° C and 80% R.H. for 48 hours. The percentage mortality of tick larvae was then recorded and compared with controls.

The controls gave a mortality of less than 5% whereas the compound of Example 1 caused greater than 50% mortality at a concentration of 100 ppm.

Use Example G

Insecticidal activity against house flies ( Musca domestica )

Aliquots of acetone solutions of test compounds at various concentrations were applied to 9 cm diameter filter papers placed in the bottom of 9 cm diameter petri dishes closed by glass lids. After evaporation of solvent, the treated surfaces, together with control treated with acetone alone, were then infested with adult houseflies, ( Musca domestica ) and held at 22° C for 24 hours. The percentage mortality of the insects was then recorded.

Less than 5% mortality resulted in the control treatments whereas the compounds of Examples 1 and 4 had an $LC_{50}$ of 300 $mg/m^2$ or less.

Use Example H

Insecticidal activity against sheep blowfly ( Lucilia sericata )

1 ml aliquots of an acetone solution containing test compound at various concentrations were applied to cotton wool dental rolls 1 cm x 2 cm, contained in glass vials (2 cm diameter x 5 cm long). After drying, the treated materials were then impregnated with 1ml of nutrient solution, infested with first instar larvae of sheep blowfly ( Lucilia sericata ), closed by a cotton wool plug and held at 25° C for 24 hours.

For the controls the mortality was <5% whereas the compound of Example 2 had an $LC_{50}$ of less than 30 ppm.

**Claims**

1. 5-Substituted 1,3,4-thiadiazole derivatives of general formula I

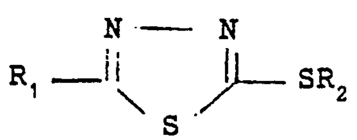

(I)

wherein

$R_1$ is $C_{1-14}$-alkyl, $C_{3-6}$-cycloalkyl or $C_{3-6}$-cycloalkylmethyl, and

$R_2$ is $C_{1-12}$-alkyl, $C_{2-12}$-alkenyl, $C_{2-12}$-alkynyl, $C_{3-6}$-cycloalkyl or $C_{3-6}$-cycloalkylmethyl, each of which is substituted one or more times by the same or different halogen.

2. Compounds according to claim 1, wherein

$R_1$ is $C_{1-4}$-alkyl or $C_{3-6}$-cycloalkyl, and

$R_2$ is halo-$C_{1-4}$-alkyl or halo-$C_{2-4}$-alkenyl.

3. Compounds according to claim 2, wherein

$R_1$ is methyl or cyclopropyl, and

$R_2$ is difluoromethyl, bromodifluoromethyl or 3,3,4-trifluoro-3-butenyl.

4. A pesticidal composition which comprises a compound claimed in any one of the preceding claims, in admixture with an agriculturally acceptable diluent or carrier.

5. A method of combating pest, especially plant parasitic nematodes, which comprises applying to the pest or its locus an effective amount of a compound claimed in any one of claims 1 to 3.

European Patent Office

# EUROPEAN SEARCH REPORT

Application Number

EP 90 25 0188

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 127 847 (HOECHST U.K. LTD.) * page 16, compound 1.8; page 77, compound, 2.8 * | 1,2 | C 07 D 285/125<br>A 01 N 43/82 |
| A,D | WO-A-8 607 590 (FMC CORP.) * claims 1,2,18,19,34,35 * | 1,4,5 | |
| A,D | EP-A-0 217 747 (CIBA-GEIGY AG) * claims 1-10,12-21 * | 1,4,5 | |
| | | | **TECHNICAL FIELDS SEARCHED (Int. Cl.5)** |
| | | | C 07 D 285/00<br>A 01 N 43/00 |

The present search report has been drawn up for all claims

| Place of search | Date of completion of the search | Examiner |
|---|---|---|
| BERLIN | 01-10-1990 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)